Europäisches Patentamt

European Patent Office

Office européen des brevets

(11) Publication number: **0 455 836 A1**

## EUROPEAN PATENT APPLICATION
### published in accordance with Art. 158(3) EPC

(21) Application number: 91900356.6

(22) Date of filing: 22.11.90

(86) International application number: PCT/JP90/01526

(87) International publication number: WO 91/07990 (13.06.91 91/13)

(51) Int. Cl.⁵: **A61K 49/00**

(30) Priority: 24.11.89 JP 303227/89
06.07.90 JP 179166/90

(43) Date of publication of application:
**13.11.91 Bulletin 91/46**

(84) Designated Contracting States:
**DE FR GB**

(71) Applicant: ARAI, Toshiyuki
**26-46, Nishifukunokawa-cho, Okazki,
Sakyo-ku
Kyoto 606(JP)**

Applicant: MORI, Kenjiro

10, Matsugasaki Sandanosa-cho
Sakyo-ku, Kyoto-shi, Kyoto 606(JP)

(72) Inventor: ARAI, Toshiyuki
26-46, Nishifukunokawa-cho, Okazki,
Sakyo-ku
Kyoto 606(JP)
Inventor: MORI, Kenjiro
10, Matsugasaki Sandanosa-cho
Sakyo-ku, Kyoto-shi, Kyoto 606(JP)

(74) Representative: Stachow, E.-W., Prof. Dr. et al
Frankenforster Strasse 135-137
W-5060 Bergisch Gladbach 1(DE)

(54) NUCLEAR MAGNETIC RESONANCE AGENT, DETECTION THEREOF, AND TOMOGRAPHY USING SAID AGENT.

(57) A nuclear magnetic resonance agent in the form of an inhalant gas containing $^{17}O$ in an amount above a natural abundance, which can be noninvasively incorporated into a living cell by the respiratory action. Since $^{17}O$ is chemically stable, this agent can be preserved long. It is possible to detect said agent incorporated into a living organism through the nuclear magnetic resonance of H or $^{17}O$ of the $H_2^{17}O$ formed by the metabolism in the cell and obtain information regarding the metabolic function of the cell and tissue perfusion quite safely on the basis of a change in the intensity of the resonance signals or by enhancing the contrast of a tomogram with the aid of the resonance signals.

FIG. 4

(Technical Field)

The present invention concerns a nuclear magnetic resonance agent for obtaining information about the metabolic function and the tissue perfusion of living cells by a nuclear magnetic resonance method and a detection method therefor, as well as a tomographic method by using the nuclear magnetic resonance agent.

(Background Art)

Nuclear magnetic resonance tomography (NMR-CT) used for a nuclear magnetic resonance diagnostic method can conduct tomograph for an inclined surface at an optional angle more easily as compared with conventional X-ray CT scanners, does not suffer from hindrance for imaging by bones or air and has extremely high safety since there is no worry of causing living body injury by X-ray exposure.

Then, tomographic images are formed, for instance, by irradiating sinusoidal signals depending on the resonance frequency of hydrogen atoms H to a living body and detecting the state of distribution of hydrogen H present as a water content $H_2{}^{17}O$ in the living body.

However, in conventional NMR-CT, since tomographic images have been obtained based on the state of distribution of hydrogen H present as the water content in the living body and the water content is present depending on the living body structure, information about the metabolism and the perfusion can not be obtained, although the living body structure can be taken.

By the way, oxygen includes, in addition to ordinary oxygen $^{16}O$, radioisotope $^{15}O$ and stable isotope $^{17}O$, in which the stable isotope $^{17}O$ has quite the same chemical property as that of ordinary oxygen $^{16}O$ and, accordingly, it gives no undesired effects when it is intaken into the living body and is served to the metabolism and the perfusion in the same manner as ordinary oxygen.

Accordingly, if the stable isotope $^{17}O$ of oxygen can be intaken into the living body and traced, information about the metabolic function and the tissue perfusion of living cells can be obtained with safety.

Since $^{17}O$ can be detected only when it is present in the form of water $H_2{}^{17}O$, the information about the metabolism and the perfusion has been obtained, for example, by administrating $^{17}O$ into the living body while carrying it on a liquid composition which is ionically equivalent with blood (artificial blood) and detecting the distribution of water content $H_2{}^{17}O$ produced by the metabolism of the living cells.

That is, in the tomography by NMR-CT or the like, since the relaxation time is different between a resonance signal issued from a water content present as $H_2{}^{17}O$ and a resonance signal issued from a water content present as $H_2{}^{16}O$ if $^{17}O$ is administrated into the living body and the concentration, the distribution or the concentration of $H_2{}^{17}O$ is detected, information about the metabolic function and the tissue perfusion can be obtained.

However, since incorporation of $^{17}O$ by means of the artificial blood into the living body has to be conducted in an invasive manner, for example, by means of injection, a burden is imposed to some extent on a subject to be examined upon conducting tomography or the like.

Further, although a liquid chemical equivalent in view of the ionic composition with the blood is used as the artificial blood for carrying $^{17}O$, the liquid chemical is nothing but an obstacle to the living body. Accordingly, metabolites contributing to the metabolic function are not utilized as they are, and not only an accurate information can not be obtained but also there may be a worry that undesired effect is given on the living body when it is administrated in a great amount and over a long period of time.

Further, such an artificial blood involves a problem that it denatures with lapse of time and is difficult to be stored for a long period of time even if it is stored under refrigeration in a state where ingredients are separated.

In view of the above, it is a subject of the present invention, upon utilizing $^{17}O$ for obtaining information on the metabolic function and the tissue perfusion of a living body, to utilize the inherent metabolic function and metabolites contributing thereto as they are, so that information about the metabolism and the perfusion of living body cells can be obtained extremely safely, $^{17}O$ can be intaken into the body in a non-invasive manner, as well as long time storage is enabled.

(Disclosure of the Invention)

For dissolving the subject, the nuclear magnetic resonance agent according to the present invention has a feature in that oxygen $^{17}O$ is incorporated into an inhalation gas at a present ratio greater than the naturally existing ratio.

Thus, when the nuclear magnetic resonance agent is inhaled, oxygen $^{17}O$ is intaken into blood by means of respiration. Then, oxygen and nutrients are delivered by way of the blood to living cells, and the living cells conduct metabolism of intaking oxygen and nutrients to form energy, as well as producing and discharging water and carbon dioxide.

Since oxygen $^{17}O$ contained in the nuclear magnetic resonance agent is oxygen having quite identical chemical property with that of ordinary oxygen $^{16}O$ except for the difference of the mass, it is served for the living body metabolism in the same manner as ordinary oxygen $^{16}O$, and $^{17}O$ is produced by a great amount in the form of $H_2^{17}O$ in a portion of each living body cell where the metabolic function is vigorous, whereas it is produced only little by little in a portion where the metabolic function is not vigorous. Accordingly, when hydrogen or oxygen in $H_2^{17}O$ is detected by the nuclear magnetic resonance, increment or decrement of $H_2^{17}O$ produced by the metabolism can be monitored due to the signal intensity and it can be judged by the observation along with time as to the degree of vigorous metabolism by way of $^{17}O$ intaken into the living body.

Further, upon cessation of the administration of the resonance agent, since a constant amount of $H_2^{17}O$ produced by metabolism is circulated through the body and the signal intensity is kept substantially constant, information about the tissue perfusion can be obtained by observing the change with time of the signal intensity.

Further, also in a case of continuously administrating the resonance agent, since a constant amount of $H_2^{17}O$ is circulated through the body after the stabilization of the signal intensity to a predetermined value, information about the tissue perfusion can be obtained in the same manner as upon cessation of the administration.

Further, when a tomographic image is formed based on the resonance signal for hydrogen or oxygen in $H_2^{17}O$, since the signal intensity is different between a portion where more $H_2^{17}O$ is produced and a portion where it is produced only in a small amount, it is possible to recognize, at a glance, a portion in which the metabolic function is vigorous or a portion in which it is not.

(Brief Explanation for the Drawing)

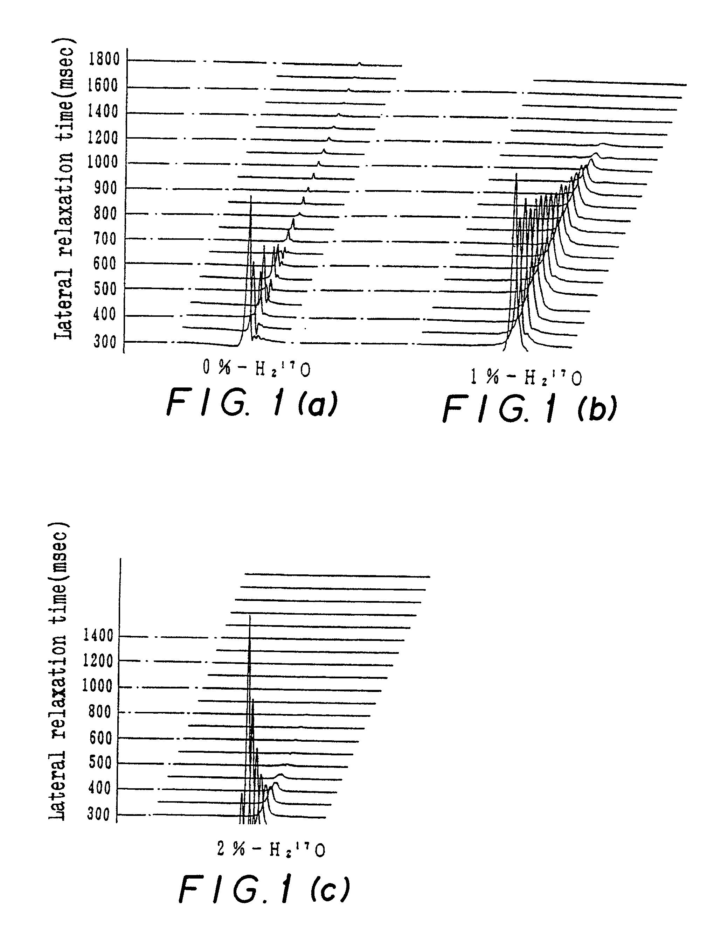

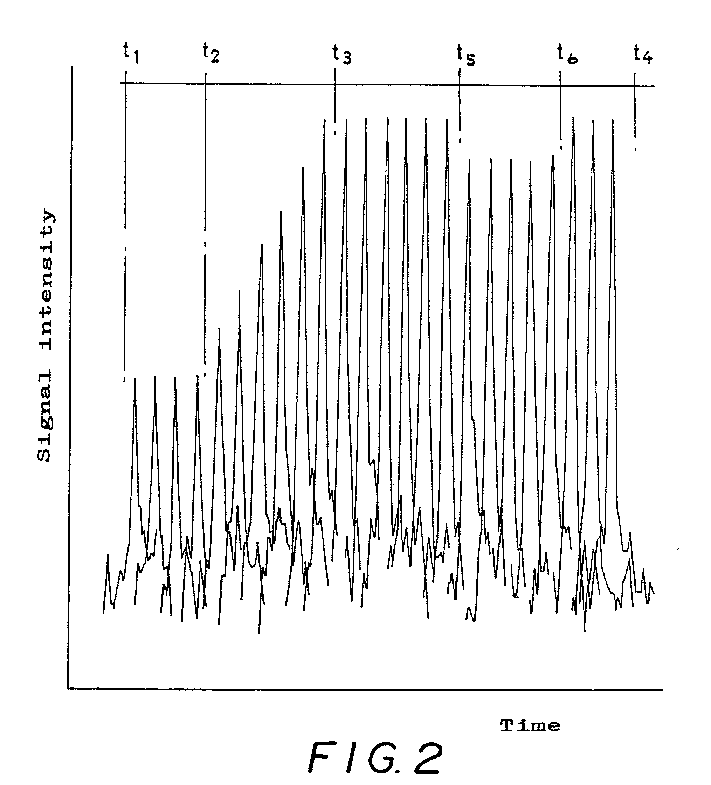

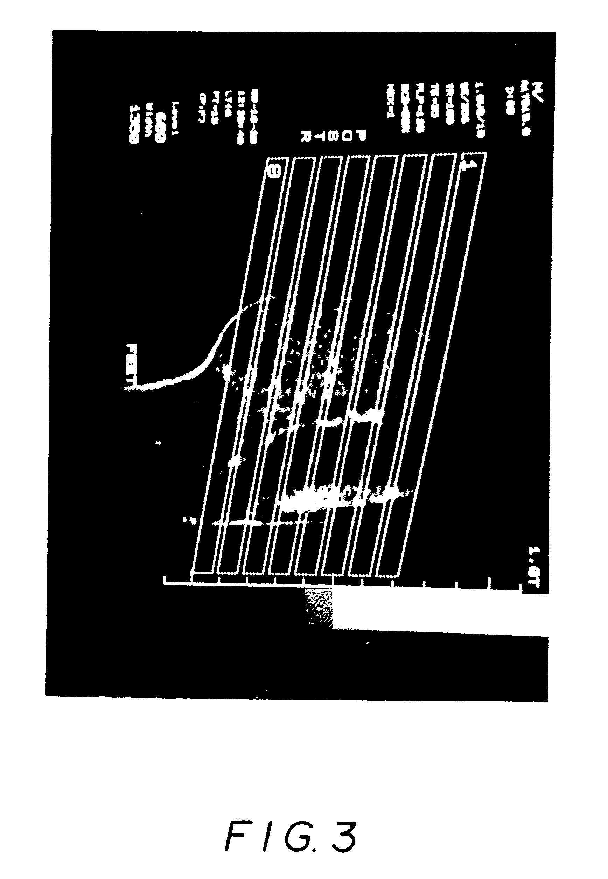

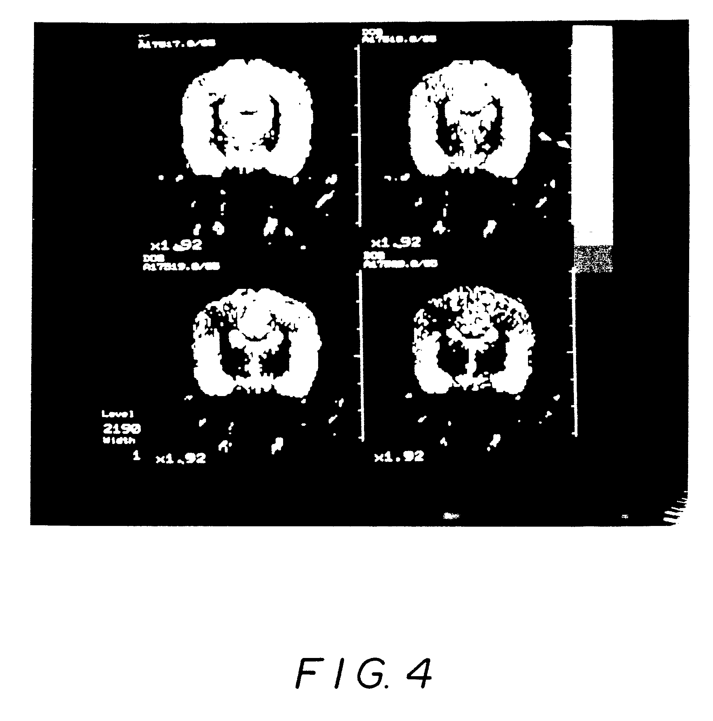

Fig. 1(a) - (c) are graphs illustrating the difference of relaxation time for resonance signals outputted from hydrogen in water in a case containing $H_2^{17}O$ by 0%, 1% and 2% respectively, Fig. 2 is a graph illustrating the change of the signal intensity upon detecting the administrated nuclear magnetic resonance agent and Fig. 3 and Fig. 4 are tomographs taken partially for the state of a living body by means of nuclear magnetic resonance tomography. (Best Mode for Practicing the Invention)

The present invention will now be explained with reference to specific examples.

The nuclear magnetic resonance agent according to the present invention is constituted, for example, by mixing a stable isotope $^{17}O$ of oxygen into an inhalation gas comprising 100% oxygen gas at a ratio greater than the naturally existing ratio (0.037%), for example, by about 40 to 50%.

The inhalation gas is supplied being charged in a gaseous or liquid form into a cylinder and adjusted for the concentration upon use, or charged in each of cylinders previously at a predetermined concentration and used with no concentration control.

Oxygen inhaled into a living body is generally intaken from a lung into blood and delivered together with nutrients to each of cells and each of the cells intakes oxygen and nutrients by the metabolic function and produces and eliminates water and carbon dioxide.

However, oxygen intaken by respiration is formed into water $H_2O$, while oxygen contained in the nutrients is formed into carbon dioxide $CO_2$.

Accordingly, in a case where $^{16}O$ and its isotope $^{17}O$ are contained in the inhalation gas, since $^{17}O$ is oxygen of quite chemically identical nature with that of $^{16}O$, it is served to metabolism in each of the cells to produce $H_2^{17}O$.

Further, in the nuclear magnetic resonance method, resonance signals for hydrogen contained in water are usually detected but, since $^{17}O$ has a magnetic spin different from $^{16}O$, the relaxation time for the resonance signals is different between hydrogen H present as $H_2^{16}O$ and hydrogen H present as $H_2^{17}O$.

Fig. 1(a) - (c) are graphs illustrating the change with time of the signal intensity for resonance signals outputted from hydrogen in water containing $H_2^{17}O$ by 0% (exactly, containing 0.037% of naturally existing ratio), 1% and 2%, respectively.

In a case where the concentration of $H_2^{17}O$ is 0%, the resonance signals are outputted still after the elapse of 1800 msec as shown in Fig. 1(a), in the case of 1% concentration, the resonance signals disappear at about 1200 msec as shown in Fig. 1(b) and, in a case of 2% concentration, the resonance signals disappear at about 900 msec as shown in Fig. 1(c).

That is, as the concentration of $H_2^{17}O$ is increased, the time up to the disappear of the resonance signal (lateral relaxation time is shortened and, accordingly, it is possible to detect hydrogen in $H_2^{17}O$ newly produced by the living body metabolism based on the lateral relaxation time to recognize the adequacy of the metabolic function of the cell.

Since a cell of vigorous metabolism produces a great amount of $H_2^{17}O$, the concentration at that portion is increased, whereas the cell with no vigorous metabolism produces $H_2^{17}O$ only little by little and, accordingly, the concentration at that portion is low.

Further, since the ordinary water content is not detected but $H_2^{17}O$ newly produced by the metabolism is detected, it can also recognize whether the metabolic function is stopped or not regarding a certain portion.

Further, when the change with time of the concentration of time $H_2{}^{17}O$ is observed, it is extremely effective as data for judging the extent of troubles occurring with respect to the metabolic function.

Furthermore, when tomography is conducted based on the nuclear magnetic resonance signals, it can be judged at a glance the site and the degree of troubles.

Further, the concentration of $^{17}O$ mixed into the inhalation gas can be selected optionally depending on the purpose of use providing that it is greater than the naturally existing ratio.

For instance, in a case of intending to shorten the detection time, higher concentration may be used so that more $^{17}O$ can be derived rapidly into the body, while lower concentration may be kept to be supplied in a case of continuously monitoring the living body function over a long period of time.

Further, although explanations have been made regarding the example to a case of using an inhalation gas in which $^{17}O$ is mixed into 100% oxygen gas, the inhalation gas is not necessarily restricted only to the 100% oxygen gas so long as it can introduce $^{17}O$ by the metabolic function into the living body and it may be an ordinary air (gas of a mixture at a ratio of nitrogen 4: oxygen 1) incorporated with $^{17}O$ and, in summary, it is only necessary that the gas contains least oxygen necessary for sustaining life and incorporated with $^{17}O$ into a non-toxicic gas.

Further, as a method of detecting the resonance agent, it is not restricted only to the case of resonating hydrogen H in $H_2{}^{17}O$ but $^{17}O$ may be resonated as described below.

Fig. 2 is a graph illustrating the change with time of signal intensity of nuclear magnetic resonance signals when an oxygen gas containing 44% of $^{17}O$ was inhaled as nuclear magnetic resonance agent for 48 mins to Japanese White Rabbits of about 3 kg body weight and increment and decrement of $^{17}O$ present as $H_2{}^{17}O$ in the brain was detected on every 8 mins before inhalation, during inhalation and after inhalation, in which the abscissa indicates the time and the ordinates indicates the signal intensity. In this case, the magnetic field is at 2 Tesla and the resonance frequency of $^{17}O$ is 11.57 MHz.

In the graphs, are shown $t_1 - t_2$ for before inhalation, $t_2 - t_3$ for during inhalation and $t_3 - t_4$ for after inhalation.

Before inhalation of the nuclear magnetic resonance agent: $t_1 - t_2$, since ordinary air is inhaled, only $H_2{}^{17}O$ at a constant amount is produced in accordance with the naturally existing ratio but the signal intensity is week and causes no intensity change.

During inhalation of nuclear magnetic resonance agent $t_2 - t_3$, the signal intensity is gradually increased from the beginning of inhalation $t_2$ to the cessation of inhalation $t_3$ and it can be seen that the amount of $H_2{}^{17}O$ produced is increased by the metabolism of cerebral cells.

As the slope for the change of the signal intensity becomes steeper, it shows that more $H_2{}^{17}O$ is produced and it can be seen that the metabolism of the cell is vigorous.

Further, as the slope becomes more moderate, the amount of $H_2{}^{17}O$ produced is decreased and it can be seen that the metabolic function of the cell is lowered.

In this way, information about the metabolic function is obtained based on the change of the signal intensity upon inhalation.

Then, after the cessation of inhalation t2, since no $H_2{}^{17}O$ is produced newly and $H_2{}^{17}O$ produced so far circulates through the living body, information regarding the tissue perfusion can be obtained.

That is, if the cell functions normally, since $H_2{}^{17}O$ present by a predetermined amount as the water content in the blood is always intaken, the signal intensity is kept at a substantially constant value.

In this case, if $H_2{}^{17}O$ is no more intaken into the cells by some or other reasons, since the signal intensity decreases abruptly, occurrence of abnormality can be observed if the signal intensity is put under monitoring.

For instance, $t_5 - t_6$ shows the signal intensity when the metabolic function of the cells is forcively suppressed by administrating pentabarbital as an anesthetic chemical (10 mg/kg) by way of intravenous injection and it can be seen that the signal intensity is apparently decreased as compared with a normal situation.

Also in a case of continuously administrating the oxygen gas, the amount of $H_2{}^{17}O$ produced is initially increased and the signal intensity is gradually increased to obtain information about the metabolic function but, after the production amount reaches a predetermined limit value and the signal intensity is stabilized at a constant value, information about the tissue perfusion can be obtained.

Accordingly, in a case of intending to obtain information only about the perfusion, since air is considered as a resonance agent containing 0.037% of $^{17}O$, information regarding the perfusion can be obtained by using ordinary air.

In this way, according to the example as described above, information about the metabolic function can be obtained due to the change of the signal intensity upon inhalation of the resonance agent and the information about the tissue perfusion can be obtained after the stabilization of the signal intensity.

The same results as described above can also be obtained by detecting, through nuclear magnetic resonance, the hydrogen H present as $H_2{}^{17}O$.

Further, Fig. 3 and Fig. 4 are tomographs for a brain which were taken based on resonance signals outputted from hydrogen H present as $H_2{}^{17}O$ when a dog of about 5 kg body weight was anesthetized with pentabarbital (about 10 mg/kg) and inhaled with 300 cc of a nuclear magnetic resonance agent comprising an oxygen gas containing 46% of a stable isotope $^{17}O$ for 10 min by spontaneous respiration and, thereafter, caused to inspire in ordinary air.

Fig. 3 is a tomograph along a saggital plane laid with a mouth being directed rightward and Fig. 4 shows tomographs along the fifth coronal plane for the portions 1 - 8 each surrounded with white lines in Fig. 3, showing photographs before inhalation, upon the elapse of 20 min, 40 min and 60 min after the cessation of inhalation of $^{17}O$ respectively, in the order of upper left, upper right, lower left and lower right portions.

In Fig. 4, images for bright portions appearing white show the cerebral tissue imaged by the resonance signals detected from hydrogen in $H_2{}^{16}O$, while the dark portions appearing black are portions from which resonance signals from hydrogen H in $H_2{}^{17}O$ produced by the cell metabolism are outputted, that is, portions in which the metabolism is taken place vigorously.

Referring to the change with time in Fig. 4, at first, a primary portion (central portion) such as thalamus controlling respiration, body temperature, etc. appears black and it can be seen that the portion functions normally even under anesthetized situation.

Then, the black portion prevails with elapse of time to the peripheral cerebral cortex portion.

In particular, in the photograph after the elapse of 60 mins, the dark portion prevails in the upper left of the brain, whereby it can be seen that the higher order function of the relevant portion of the brain of the dog as a subject to be examined, awoke from anesthetization and began to function normally and recovered sufficiently.

In this way, it is possible to discriminate portions with vigorous function from portions without such function, for example, by taking tomographs for the brain by NMR-CT using the resonance agent according to the present invention or to judge the extent of the function by monitoring the change with elapse of time.

(Industrial Applicability)

As has been described above according to the present invention, since an inhalation gas containing $^{17}O$ by more than the naturally existing ratio us used as the nuclear magnetic resonance agent, the resonance agent can be intaken by way of respiration into the body in a non-invasive manner.

Further, since $^{17}O$ is biochemically equivalent with $^{16}O$, inherent metabolic function and perfusion function can be utilized as they are without giving undesired effects on the living body and, in addition, since $^{17}O$ is contained at a ratio greater than the naturally existing ratio, information about the metabolic function and the tissue perfusion of living body cells can be obtained with safety, by detecting hydrogen or oxygen in $H_2{}^{17}O$.

Further, since $^{17}O$ is stable, it can be stored for a long period of time without denaturation.

## Claims

1. A nuclear magnetic resonance agent wherein oxygen $^{17}O$ is incorporated into an inhalation gas at a presence ratio greater than the naturally existing ratio.

2. A nuclear magnetic resonance agent as defined in claim 1, wherein the nuclear magnetic resonance agent is a liquefied gas.

3. A method of detecting a nuclear magnetic resonance agent, which comprises intaking a nuclear magnetic resonance agent in which oxygen $^{17}O$ is incorporated into an inhalation gas at a presence ratio greater than the naturally existing ratio into living cells by way of respiration, and causing hydrogen H in $H_2{}^{17}O$ produced by the metabolic function of the living cells to nuclear magnetic resonance, thereby detecting the resonance agent based on resonance signals thereof.

4. A method of detecting a nuclear magnetic resonance agent, which comprises intaking a nuclear magnetic resonance agent containing oxygen $^{17}O$ into at a presence ratio greater than the naturally existing ratio into living cells and causing oxygen $^{17}O$ produced by the metabolic function of the living cells to nuclear magnetic resonance, thereby detecting the resonance agent based on resonance signals thereof.

5. A tomographic method based on nuclear magnetic resonance, which comprises intaking a nuclear magnetic resonance agent in which oxygen $^{17}O$ is incorporated into an inhalation gas at a presence ratio greater than the naturally existing ratio into living cells by way of respiration, and causing hydrogen H or oxygen

$^{17}O$ in $H_2{}^{17}O$ produced by the metabolic function of the living cells to nuclear magnetic resonance, thereby forming tomographic images based on resonance signals thereof.

FIG. 1 (a) 0 % - H₂¹⁷O

FIG. 1 (b) 1 % - H₂¹⁷O

FIG. 1 (c) 2 % - H₂¹⁷O

FIG. 2

FIG. 3

*F I G. 4*

# INTERNATIONAL SEARCH REPORT

International Application No PCT/JP90/01526

## I. CLASSIFICATION OF SUBJECT MATTER (if several classification symbols apply, indicate all) 6

According to International Patent Classification (IPC) or to both National Classification and IPC

Int. Cl⁵  A61K49/00

## II. FIELDS SEARCHED

Minimum Documentation Searched 7

| Classification System | Classification Symbols |
|---|---|
| IPC | A61K49/00 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| P | WO, A, 9001953 (Gupte, Pradeep), March 8, 1990 (08. 03. 90) & AU, A, 8940665 & EP, A, 386199 | 1-2 |
| P | Biochemical and Biophysical Research Communication, Vol. 169, No. 1, (1990), Arai, Toshiyuki et al. [ Cerebral oxygen utilization analyzed by the use of oxygen-17 and its nuclear magnetic resonance ] p. 153-158 | 1-2 |
| A | Studio Biophysical, Vol. 91, No. 1, (1982), Burgar, M.I., [ Hydration role of water in biological systems as determined by oxygen-17 NMR.] p. 29-36 | |
| A | Bull. Magn. Reson., Vol. 2, No. 1-4, (1981), Fiat, D. et al. [ Oxygen-17 nuclear magnetic resonance and its biological applications ] p. 18-21 | 1-2 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| February 14, 1991 (14. 02. 91) | March 4, 1991 (04. 03. 91) |

| International Searching Authority | Signature of Authorized Officer |
|---|---|
| Japanese Patent Office | |

Form PCT ISA 210 (second sheet) (January 1985)

## FURTHER INFORMATION CONTINUED FROM THE SECOND SHEET

| | | |
|---|---|---|
| Y | Invest. Radiol., Vol. 23, suppl. No. 1, (1988), Hopkins, Amos L. et al. [Oxygen-17 contrast agents Fast imaging techniques.] S. 240-242 | 1-2 |
| A | Magn. Reson. Med., Vol. 4, No. 4, (1987), Hopkins, A. L. et al. [Oxygen-17 compounds as potential NMR T2 contrast agents: enrichment effects of 17-O-water on protein solutions and living tissues], p. 399-403 | 1-2 |
| A | Bull. Magn. Reson., Vol. 6, No. 1-2, (1984), Fiat, Daniel et al. | 1-2 |

## V [X] OBSERVATIONS WHERE CERTAIN CLAIMS WERE FOUND UNSEARCHABLE [1]

This international search report has not been established in respect of certain claims under Article 17(2) (a) for the following reasons:

1 [X] Claim numbers 3-5 because they relate to subject matter not required to be searched by this Authority, namely:

The invention as set forth in each of the claims 3 to 5 of the instant application relates to a method of diagnosing a human or animal body.

2 [ ] Claim numbers , because they relate to parts of the international application that do not comply with the prescribed requirements to such an extent that no meaningful international search can be carried out, specifically:

3 [ ] Claim numbers , because they are dependent claims and are not drafted in accordance with the second and third sentences of PCT Rule 6.4(a).

## VI [ ] OBSERVATIONS WHERE UNITY OF INVENTION IS LACKING [2]

This International Searching Authority found multiple inventions in this international application as follows:

1 [ ] As all required additional search fees were timely paid by the applicant, this international search report covers all searchable claims of the international application

2 [ ] As only some of the required additional search fees were timely paid by the applicant, this international search report covers only those claims of the international application for which fees were paid, specifically claims:

3 [ ] No required additional search fees were timely paid by the applicant. Consequently, this international search report is restricted to the invention first mentioned in the claims; it is covered by claim numbers:

4 [ ] As all searchable claims could be searched without effort justifying an additional fee, the International Searching Authority did not invite payment of any additional fee.

Remark on Protest

[ ] The additional search fees were accompanied by applicant's protest.

[ ] No protest accompanied the payment of additional search fees.

Form PCT/ISA/210 (supplemental sheet (2)) (January 1985)

| | | |
|---|---|---|
| FURTHER INFORMATION CONTINUED FROM THE SECOND SHEET | | |
| Y | Invest. Radiol., Vol. 23, suppl. No. 1, (1988), Hopkins, Amos L. et al. [Oxygen-17 contrast agents Fast imaging techniques.] S. 240-242 | 1-2 |
| A | Magn. Reson. Med., Vol. 4, No. 4, (1987), Hopkins, A. L. et al. [Oxygen-17 compounds as potential NMR T2 contrast agents: enrichment effects of 17-O-water on protein solutions and living tissues], p. 399-403 | 1-2 |
| A | Bull. Magn. Reson., Vol. 6, No. 1-2, (1984), Fiat, Daniel et al. | 1-2 |

**V.[X] OBSERVATIONS WHERE CERTAIN CLAIMS WERE FOUND UNSEARCHABLE** [1]

This international search report has not been established in respect of certain claims under Article 17(2) (a) for the following reasons:

1 [X] Claim numbers **3-5** , because they relate to subject matter not required to be searched by this Authority, namely:

The invention as set forth in each of the claims 3 to 5 of the instant application relates to a method of diagnosing a human or animal body.

2.☐ Claim numbers    , because they relate to parts of the international application that do not comply with the prescribed requirements to such an extent that no meaningful international search can be carried out, specifically:

3.☐ Claim numbers    , because they are dependent claims and are not drafted in accordance with the second and third sentences of PCT Rule 6.4(a).

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)

International Application No **PCT/JP90/01526**

---

**FURTHER INFORMATION CONTINUED FROM THE SECOND SHEET**

[ Biophysical applications of oxygen-17
NMR.], p. 30-37

---

**V. ⬚ OBSERVATIONS WHERE CERTAIN CLAIMS WERE FOUND UNSEARCHABLE** [1]

This international search report has not been established in respect of certain claims under Article 17(2) (a) for the following reasons:

1. ⬚ Claim numbers        because they relate to subject matter not required to be searched by this Authority, namely:

2. ⬚ Claim numbers        , because they relate to parts of the international application that do not comply with the prescribed requirements to such an extent that no meaningful international search can be carried out, specifically:

3. ⬚ Claim numbers        , because they are dependent claims and are not drafted in accordance with the second and third sentences of PCT Rule 6.4(a).

---

**VI. ⬚ OBSERVATIONS WHERE UNITY OF INVENTION IS LACKING** [2]

This International Searching Authority found multiple inventions in this international application as follows:

1. ⬚ As all required additional search fees were timely paid by the applicant, this international search report covers all searchable claims of the international application

2. ⬚ As only some of the required additional search fees were timely paid by the applicant, this international search report covers only those claims of the international application for which fees were paid, specifically claims:

3. ⬚ No required additional search fees were timely paid by the applicant. Consequently, this international search report is restricted to the invention first mentioned in the claims; it is covered by claim numbers:

4. ⬚ As all searchable claims could be searched without effort justifying an additional fee, the International Searching Authority did not invite payment of any additional fee.

Remark on Protest

    ⬚ The additional search fees were accompanied by applicant's protest.

    ⬚ No protest accompanied the payment of additional search fees.

---

Form PCT/ISA/210 (supplemental sheet (2)) (January 1985)